# EUROPÄISCHE PATENTANMELDUNG

(11) **EP 1 208 802 A1**
(43) Veröffentlichungstag der Anmeldung: **29.05.2002**
(21) Anmeldenummer: 01104486.4
(22) Anmeldetag: 07.03.2001
(51) Int. Cl.: A61B 17/22, A61N 7/00

(54) **Ultraschallgerät zur Behandlung septischer Wunden**

(30) Priorität: 20.11.2000 DE 20019711 U
(71) Anmelder: Söring GmbH, 25451 Quickborn (DE)
(72) Erfinder: Söring, Jörg, 25488 Holm (DE); Söring, Holger, 25451 Quickborn (DE)

(57) **Zusammenfassung**

Die vorliegende Erfindung betrifft ein Ultraschall-Gerät zur Behandlung septischer Wunden, welches aus einem Handstück (1) und daran befestigter Sonotrode (7) besteht. Durch einen innerhalb des Handstücks angeordneten Kanal (10) kann während des Einsatzes des Gerätes Behandlungsflüssigkeiten wie Kochsalzlösung oder heilmedizinische Mittel dem Sonotrodenkopf zugeführt werden.

## Beschreibung

Gegenstand der Erfindung ist ein Ultraschall-Gerät zur Behandlung von septischen Wunden, z.B. von sog. offenen Beinen oder anderen insbesondere bakteriell verseuchten Wunden, welches aus einem mit einem Ultraschallgenerator und einem Flüssigkeitsspeicher verbundenen Handstück und einer daran befestigten Sonotrode besteht.

Die bisher bekannten Behandlungsformen von septischen Wunden sind nicht nur für den Patienten sehr schmerzhaft, sondern außerdem sehr zeitaufwendig und somit kostenintensiv. Ein Heilungserfolg ist bei diesen herkömmlichen Behandlungsmethoden nicht garantiert.
In besonders schweren Fällen ist auch eine Transplantation von Hautteilen von anderen Körperstellen nötig.

Der vorliegenden Erfindung liegt die Aufgabe zugrunde, ein Ultraschall-Gerät für die Behandlung von septischen Wunden zu gestalten, welches für ein kostengünstiges Behandlungsverfahren sorgt und dieses Verfahren für den Patienten weniger schmerzhaft ist.

Gelöst wird diese Aufgabe durch die besondere Gestaltung der Sonotrode eines Ultraschall-Gerätes, dergestalt, dass innerhalb der Sonotrode ein Kanal zum Zuführen von Kochsalzlösung und gegebenenfalls von heilmedizinischen Mitteln wie Heparin zur Behandlungsfläche des je nach Behandlungsart unterschiedlich gestalteten Sonotrodenkopfes vorgesehen ist.

Besondere Ausgestaltungen des Erfindungsgegenstandes sind den Unteransprüchen zu entnehmen.

Um die Behandlungsverfahren zu optimieren, wurden Sonotrodenkopf und Behandlungsfläche der Sonotrode in sehr unterschiedlicher den verschiedenen Körper- und Wundformen angepasster Form gestaltet. In der Anwendung dieses sog. Ultraschallverfahrens wird die bakterizide, reinigende und massierende Wirkung des Ultraschalls ausgenutzt.
Der Einsatz von Flüssigkeitsaerosolen wird der direkte Kontakt der Behandlungsfläche des Ultraschallkopfes mit den schmerzempfindlichen Wundbereichen vermieden. Durch die Anwendung des Ultraschalls wird die Mikrozirkulation angeregt, d.h. durch die massierende Wirkung des Ultraschalls werden die infizierten Zellen zerstört und somit die Wundoberfläche gereinigt und die Wunde verheilt bei regelmäßiger Anwendung der Ultraschallbehandlung innerhalb kurzer Zeit.
Durch den innerhalb der Sonotrode vorgesehenen Kanal können neben der Kochsalzlösung auch heilmedizinische Mittel wie Heparin, Antibiotika etc. eingesetzt werden, was sich insbesondere bei schwer zugänglichen Infektionsgebieten empfiehlt. Eine solche Ultraschall-Behandlung ist insbesondere dann hilfreich, wenn durch Injektionen im Wundgebiet keine Besserung erreicht wurde. Der Einsatz von Flüssigkeiten hat zusätzlich noch den Vorteil, dass hierdurch die während der Behandlung entstehende Wärme vom Sonotrodenkopf abgeführt wird.

Wie schon erwähnt, sind die Sonotrodenköpfe des Erfindungsgegenstandes sehr unterschiedlich geformt. Die Form des Sonotrodenkopfes und der Behandlungsfläche ist sehr stark abhängig von Lage und Form der Wunde, damit eine optimale Ausnutzung der Schallenergie gewährleistet ist.

Anhand von Zeichnungen verschiedener Ausführungsformen soll der Erfindungsgegenstand näher erläutert werden. Es zeigt:
- Fig. 1: zeigt einen teilweisen Längsschnitt durch ein Handstück mit auswechselbarem Sonotrodenkopf;
- Fig. 2: zeigt eine Sonotrode mit schräg zur Längsachse geformter Behandlungsfläche;
- Fig. 3a: zeigt eine Sonotrode mit einer Behandlungsfläche in Form einer Schraubendreherklinge;
- Fig. 3b: zeigt eine Ansicht auf die Stirnseite der Sonotrode aus Fig. 3a;
- Fig. 4: zeigt eine Sonotrode mit einer tellerartigen Behandlungsfläche;
- Fig. 5: zeigt eine Sonotrode mit einer kugelförmigen Behandlungsfläche;

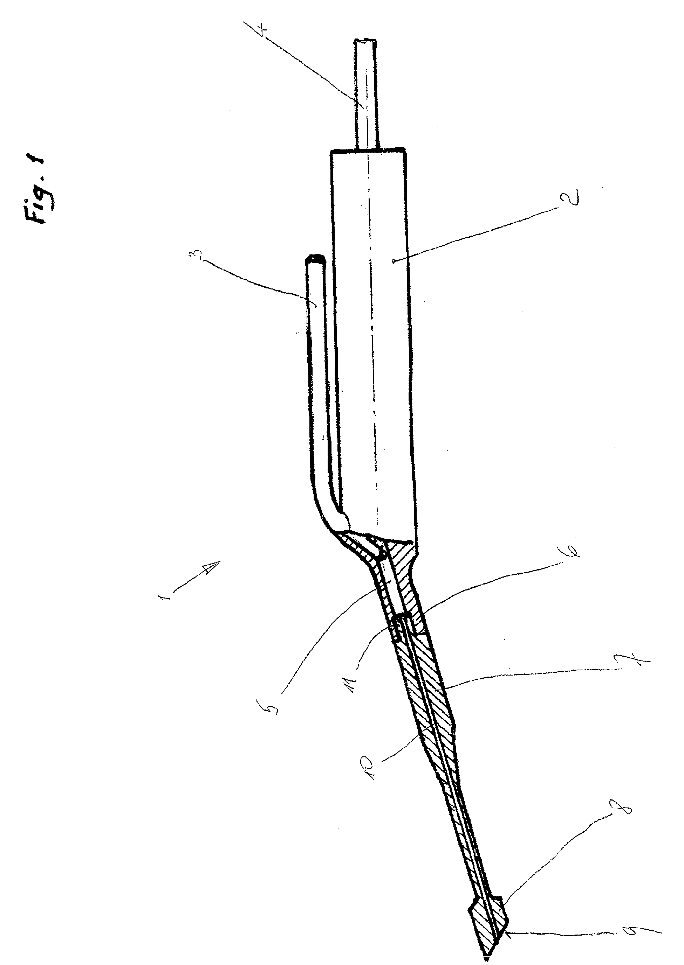

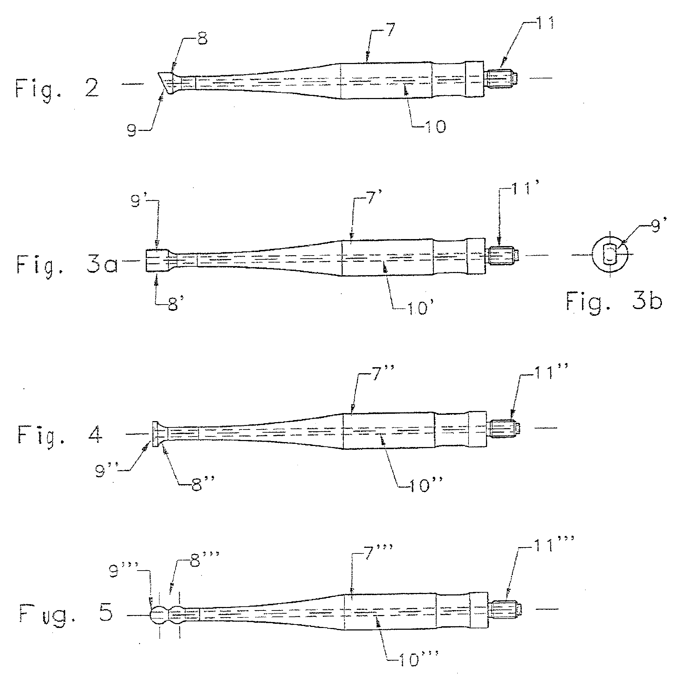

Das in Figur 1 dargestellte Ultraschall-Gerät besteht aus einem Handstück (1) mit einem Griffbereich (2), einem Anschlussrohr (3) zum Anschluss an einen Versorgungsschlauch, durch den Kochsalzlösung und medizinische Heilmittel wie Heparin, Antibiotika etc. durch den Kanal (5) zur Sonotrode (7) geleitet werden. Die Ultraschall-Energie wird durch die Anschlussleitung zum Ultraschall-Generator (4) in an sich bekannter Weise dem Handstück (1) zugeführt.
Das Handstück (1) ist mit einer Sonotroden-Aufnahme (6) ausgerüstet, die ein Gewinde zum Einschrauben der Sonotrode (7,7',7",7"') aufweist. Die jeweilige Sonotrode (7,7',7",7"') ist mit einem dem Gewinde des Handstückes entsprechenden Befestigungsgewinde (11,11',11",11"') versehen.
Durch den Kanal (10,10',10",10"') wird die der Behandlungsfläche (9,9',9",9"') zuzuführende Flüssigkeit geleitet.
Die behandlungsseitigen Enden der Sonotroden (7,7',7",7"') können mit sehr unterschiedlichen Sonotrodenköpfen (8,8',8",8"') ausgestattet sein. So zeigt z.B. Figur 2 einen Sonotrodenkopf (8) mit zylindrischer Außenform. Die Behandlungsfläche (9) ist vorzugsweise schräg zur Längsachse abgefräst und poliert. Es ist auch denkbar, diese Fläche konkav auszuführen, um eine gezielte Verteilung der Flüssigkeit zu bewirken.

Figur 3a zeigt eine Längsansicht einer Sonotrode (7') mit einem Befestigungsgewinde (11'), einem Kanal (10') und einem Sonotrodenkopf (8'), der die Form einer Schraubendreherklinge hat, so dass - wie aus Figur 3b zu ersehen ist- die Behandlungsflächen (9') seitlich liegen. Bei dieser besonderen Gestaltung ist es auch denkbar, dass der Kanal (10') stimseitig am Sonotrodenkopf verschlossen und stattdessen mitten durch die Behandlungsflächen (9') eine Querbohrung vorgesehen ist.

In Figur 4 ist die Sonotrode (7") mit einem tellerförmigen Sonotrodenkopf (8") ausgestattet. Auch diese Sonotrode wird mittels des Befestigungsgewindes (11") mit dem Handstück (1) verbunden.

Durch den Kanal (10") wird die Behandlungsflüssigkeit der Behandlungsfläche (9") zugeführt. Diese Behandlungsfläche (9") kann konkav gestaltet sein. Durch eine solche Form einer Behandlungsfläche kann die Zerstäubungsleistung der Flüssigkeiten noch erhöht werden. Mit der Flüssigkeitszerstäubung erfolgt gleichzeitig eine Ableitung der durch Ultraschall erzeugten Wärme innerhalb des Applikators und an den abstrahlenden Grenzflächen.

Bei der in Figur 5 dargestellten Sonotrode (7"') ist der Sonotrodenkopf (8"') kugelförmig gestaltet. Mit dem Befestigungsgewinde (11"') kann die Sonotrode (7"') mit dem Handstück (1) verbunden werden. Die kugelförmige Behandlungsfläche (9"') wird durch den Kanal (10"') mit Behandlungsflüssigkeit versorgt. Eine solche kugelförmig gestaltete Behandlungsfläche erlaubt ein punktgenaues Ansetzen der Sonotrode (7"') im Wundbereich. Der Sonotrodenkopf kann -wie dargestellt- aus zwei aneinander gesetzten Kugeln oder auch aus einer oder drei und mehr Kugeln bestehen. Die Zahl der aneinander gesetzten Kugel beeinflusst Intensität und Verlauf der Ultraschallschwingungen.

Außer den in den Figuren 2 bis 5 dargestellten Formen von Sonotrodenköpfen sind selbstverständlich auch noch andere, speziellen Anforderungen entsprechend gestaltete Sonotrodenkopfformen -z.B. eine löffelförmige Gestaltung- denkbar.

Für alle dargestellten und beschriebenen Formen von Sonotrodenköpfen gilt, dass mit der Flüssigkeitszerstäubung gleichzeitig eine Anleitung der durch Ultraschall erzeugten Wärme innerhalb des Applikators und an den abstrahlenden Grenzflächen erfolgt. Evtl. erforderliche Berührungskontakte mit dem Wundgebiet können durch den vorhandenen Flüssigkeitsnebel und/oder den oberflächigen Flüssigkeitsfilm schmerzarm ausgeführt werden. Die bakteriziden Effekte des Ultraschalls wirken sterilisierend innerhalb der behandelten Zone. Während das Auftreten von Flüssigkeitskavitation zur Reinigung oberflächiger Gewebezonen beiträgt, regt die durch den hohen Schallwechseldruck forcierte Mikromassage die Mikrozirkulation im Behandlungsgebiet an.

### Positionsnummernliste:

- 1: - Handstück
- 2: - Griffbereich
- 3: - Anschlussleitung
- 4: - Anschluss zum Ultraschallgenerator
- 5: - Zuführkanal
- 6-: - Sonotroden-Aufnahme
- 7, 7', 7", 7"': - Sonotrode
- 8, 8', 8", 8"': - Sonotrodenkopf
- 9, 9', 9", 9"': - Behandlungsfläche
- 10, 10', 10", 10"': - Kanal
- 11, 11', 11", 11"': - Befestigungsgewinde

## Patentansprüche

1. Ultraschall-Gerät zur Behandlung von septischen Wunden, welches aus einem mit einem Ultraschallgenerator und einem Flüssigkeitsspeicher verbundenen Handstück (1) und einer daran befestigten Sonotrode (8) besteht, **dadurch gekennzeichnet, dass** innerhalb der Sonotrode (8, 8', 8", 8"') ein Kanal (10, 10', 10", 10"') zum Zuführen von Behandlungsflüssigkeit wie Kochsalzlösung und gegebenenfalls von heilmedizinischen Mitteln wie Heparin, Antibiotika etc. zur Behandlungsfläche (9, 9', 9", 9"') des der erforderlichen Behandlungsart angepassten Form des Sonotrodenkopfes (8, 8', 8", 8"') vorgesehen ist.

2. Ultraschall-Gerät zur Behandlung von septischen Wunden nach Anspruch 1, **dadurch gekennzeichnet, dass** der Sonotrodenkopf (8) aus einem zylindrisch geformten Teil besteht, dessen Behandlungsfläche (9) schräg zur Längsachse der Sonotrode geformt ist.

3. Ultraschall-Gerät zur Behandlung von septischen Wunden nach Anspruch 2, **dadurch gekennzeichnet, dass** die Behandlungsfläche (9) konkav gestaltet ist.

4. Ultraschall-Gerät zur Behandlung von septischen Wunden nach Anspruch 1, **dadurch gekennzeichnet, dass** der Sonotrodenkopf (8') die Form einer mit parallelen Seitenflächen versehenen Schraubendreherklinge hat.

5. Ultraschall-Gerät zur Behandlung von septischen Wunden nach Anspruch 1, **dadurch gekennzeichnet, dass** der Sonotrodenkopf (8") tellerförmig gestaltet ist.

6. Ultraschall-Gerät zur Behandlung von septischen Wunden nach Anspruch 5, **dadurch gekennzeichnet, dass** die Behandlungsfläche (9") des Sonotrodenkopfes (8") konkav gestaltet ist.

7. Ultraschall-Gerät zur Behandlung von septischen Wunden nach Anspruch 1, **dadurch gekennzeichnet, dass** der Sonotrodenkopf (8"') und damit die Behandlungsfläche (9"') kugelförmig geformt ist.

8. Ultraschall-Gerät zur Behandlung von septischen Wunden nach Anspruch 7, **dadurch gekennzeichnet, dass** der Sonotrodenkopf aus einer oder mehreren aneinander gesetzten Kugeln besteht.

9. Ultraschall-Gerät zur Behandlung von septischen Wunden nach einem oder mehreren der vorhergehenden Ansprüche, **dadurch gekennzeichnet, dass** die Behandlungsflächen (9, 9', 9", 9"') der Sonotrodenköpfe (8, 8', 8", 8"') poliert sind.

10. Ultraschall-Gerät zur Behandlung von septischen Wunden nach einem oder mehreren der vorhergehenden Ansprüche, **dadurch gekennzeichnet, dass** die Sonotroden (7, 7', 7", 7"') aus einem verschleißfesten, autoklavierbaren Material bestehen.
